Europäisches Patentamt

European Patent Office

Office européen des brevets

(19)

(11) **EP 0 891 935 A1**

(12) **EUROPÄISCHE PATENTANMELDUNG**

(43) Veröffentlichungstag:
20.01.1999 Patentblatt 1999/03

(51) Int Cl.⁶: **B65G 65/46**, B65B 1/44

(21) Anmeldenummer: **98202206.3**

(22) Anmeldetag: **24.10.1991**

(84) Benannte Vertragsstaaten:
**AT BE CH DE DK ES FR GB GR IT LI LU NL SE**

(30) Priorität: **13.11.1990 DE 4036066**

(62) Dokumentnummer(n) der früheren Anmeldung(en)
nach Art. 76 EPÜ:
**91118099.0 / 0 485 772**

(71) Anmelder: **BÜHLER AG**
**9240 Uzwil (CH)**

(72) Erfinder: **Die Erfindernennung liegt noch nicht
vor**

Bemerkungen:
Diese Anmeldung ist am 20 - 06 - 1998 als
Teilanmeldung zu der unter INID-Kode 62
erwähnten Anmeldung eingereicht worden.

(54) **Regelvorrichtung für ein Kompaktierwerk**

(57)    Eine Regelvorrichtung weist eine Stelleinrichtung (10, 11, 66; 73, 166; 173, 266; 366, 86) zur Veränderung der Schüttdichte am Ausgang eines Kompaktierwerkes (11) für das Mahlwerk auf. Dazu ist an den Ausgang des Kompaktierwerkes eine Messeinrichtung für Volumen und Gewicht einer Probe angeschlossen ist, deren Messignale einer Recheneinrichtung (45) zur Errechnung der Schüttdichte zuführbar sind. Die Ausgangssignale dieser Recheneinrichtung (64) sind einer Regeleinrichtung (64) zum Vergleich mit einem Sollwert übermittelbar, an welche die Stelleinrichtung (10, 11, 66; 73, 166; 173, 266; 366, 86) zur Veränderung der Schüttdichte angeschlossen ist.

Fig.3

**Beschreibung**

Die Erfindung bezieht sich auf eine Regelvorrichtung nach dem Oberbegriffe des Anspruches 1.

Bei Walzwerken zum Mahlen von Schüttgütern ist es bekannt, das gemahlene Schüttgut anschliessend zu verpacken. Die Verpackungen sind im allgemeinen gleichmässiger Grösse und sollen auch gleichmässig gefüllt werden. Deshalb ist für das Produkt eines solchen Mahlwerkes oft eine Kompaktierungseinrichtung vorgesehen, die durch Sicherung einer einheitlichen Schüttdichte für ein gleichmässiges Aussehen der fertiggestellten Verpackung sorgt. Dies ist vor allem bei Kaffee von Bedeutung, aber gegebenenfalls bei anderen Konsumgütern.

Trotz der Kompaktierungseinrichtung kann es aber dennoch zu ungleichmässiger Befüllung dann kommen, wenn etwa das Produkt des Mahlwerkes über die Zeit Unterschiede in der Grösse oder Konsistenz aufweist.

Der Erfindung liegt daher die Aufgabe zugrunde, eine gleichmässigere Schüttdichte zu erzielen. Diese Aufgabe wird durch die kennzeichnenden Merkmale des Anspruches 1 gelöst.

Weitere Einzelheiten ergeben sich anhand der nachfolgenden Beschreibung eines in der Zeichnung schematisch dargestellten Ausführungsbeispieles. Darin zeigen:

Fig. 1    eine perspektivische Darstellung einer mit einer erfindungsgemässen Messeinrichtung zur Regelung ausgerüsteten Mühle, von der

Fig. 2    einen Schnitt nach der Linie II-II der Fig. 1 und

Fig. 3    die Messeinrichtung im Detail im Schnitt nach der Linie III-III der Fig. 1 veranschaulicht, wogegen an Hand der

Fig. 4a bis 4c drei Varianten zur Regelung der Schüttdichte er läutert werden, wobei Fig. 4a einen vergrösserten Schnitt durch die Kompaktiereinrichtung, ähnlich der Schnittführung der Fig. 2, darstellt und die Fig. 4b und 4c zwei Ausführungen einer Klappenbetätigung am Auslauf der Kompaktiereinrichtung in einem teilweisen Längsschnitt zeigen; und

Fig. 5    ein Diagramm zur Veranschaulichung der Korngrössenverteilungen der Beispiele nach der beschriebenen Tabelle;

Fig. 6    zeigt die zugehörige Partikelgrössenverteilung.

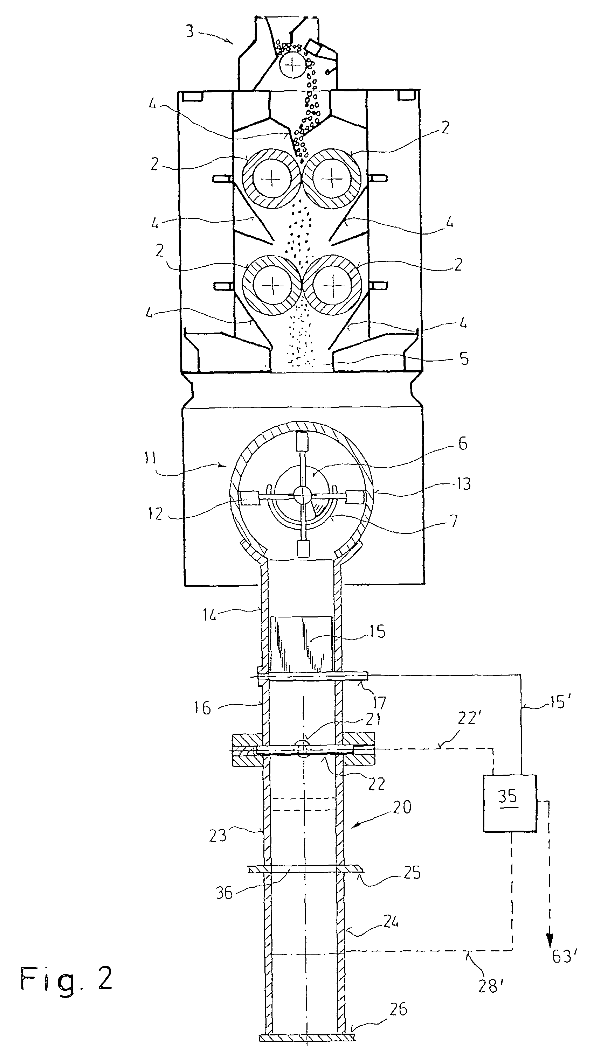

Eine Walzenmühle 1 ist zum Trockenvermahlen von Schüttgut und insbesondere von relativ sprödem oder versprödetem Gut, z.B. von gerösteten Nahrungsmitteln, wie Kaffee, ausgebildet. Sie besitzt zu diesem Zwecke wenigstens ein Paar, vorzugsweise wenigstens zwei Paare von Walzen 2. Wie Fig. 2 zeigt, wird diesen Walzen 2 Schüttgut über eine Speiseeinrichtung 3 direkt in den Walzenspalt des ersten Paares von Walzen 2 zugeführt, die das Schüttgut vorvermahlen, und anschliessend einem zweiten Walzenpaar 2, wobei Zufuhrflächen 4 dafür sorgen, dass das trockene Schüttgut in den jeweils darunterliegenden Walzenspalt bzw. in einen Auslauf 5 gelangen.

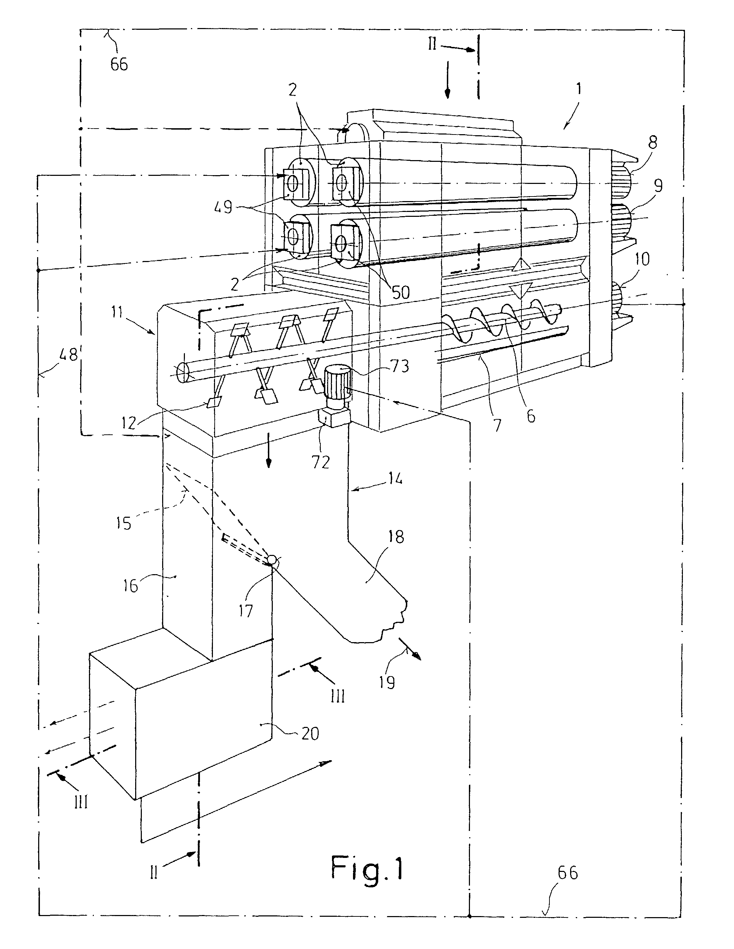

Unterhalb des Auslaufes 5 ist eine, am besten aus Fig. 1 erkennbare Sammelschnecke 6 in einem rinnenförmigen Schneckengehäuse 7 angeordnet. Während den Walzenpaaren 2 jeweils eigene Antriebe 8 und 9 zugeordnet sind, besitzt die Sammelschnecke 6 einen gesonderten Motor 10, der über ein (nicht dargestelltes) Reduziergetriebe die Schnecke 6 antreibt.

Wie ferner aus Fig. 1 ersichtlich ist, ist gleichachsig mit der Schnecke 6 ein Homogenisier- und Kompaktierwerk 11 in einem eigenen Gehäuse angeordnet, das zur Durchmischung und Schüttdichtenveränderung mit Paddelarmen 12 versehen ist, die etwa radial abstehen. Dieses Homogenisier- und Kompaktierwerk 11 dient der Austragung des gemahlenen Produktes, wobei es sich versteht, dass die Ausbildung unterhalb der Walzen 2 an sich beliebig sein könnte, obwohl gerade für die, ebenfalls beabsichtigte, Messung der Durchschnittsmahlfeinheit, aber auch zur Schüttdichtenbestimmung die Durchmischung von besonderem Vorteil ist. Es sei jedoch erwähnt, dass an Stelle der gezeigten Werkzeuge auch Auflösungswerkzeuge zur Auflösung von anfallenden Agglomeraten vorgesehen sein können.

Wie besonders aus Fig. 2 ersichtlich ist, sitzt das Kompaktierwerk 11 in einem die Paddelarme 12 relativ eng umgebenden Gehäuse 13. An dieses Gehäuse 13 schliesst nach unten zu eine, ganz allgemein aus Fig. 1 ersichtliche Rohrleitung 14 oder von einer eckigen auf eine runde Querschnittsform überleitende Trimelle 14. Von dieser Rohrleitung 14 zweigt eine normalerweise durch eine Klappe 15 verschlossene Probenehmerleitung 16 ab. Die Klappe 15 (an ihrer Stelle kann auch ein anderer Probenehmer vorgesehen sein) ist um eine Achse 17 schwenkbar, die in zeitlichen Abständen über eine nicht dargestellte Betätigungseinrichtung derart schwenkbar ist, dass der Rohrleitungsabschnitt 16 freigegeben wird. Während also normalerweise das fertig gemahlene Gut über einen Rohrleitungsabschnitt 18 im Sinne des Pfeiles 19 an einen Silo, eine Absackeinrichtung oder einen Förderer abgegeben wird, fällt ein Teil des aus der Homogenisiereinrichtung 11 tretenden Gutes bei entsprechender Stellung der Klappe 15 in den Probenehmerschacht 16. Die Betätigung der Klappe 15 wird im allgemeinen über ein Zeitglied in regelmässigen Zeitabständen erfolgen, doch kann es für manche Anwendungsfälle bevorzugt sein, einen Zufallsgenerator einzusetzen, über den die Klappe 15 in unregelmässigen Zeitabständen betätigt wird.

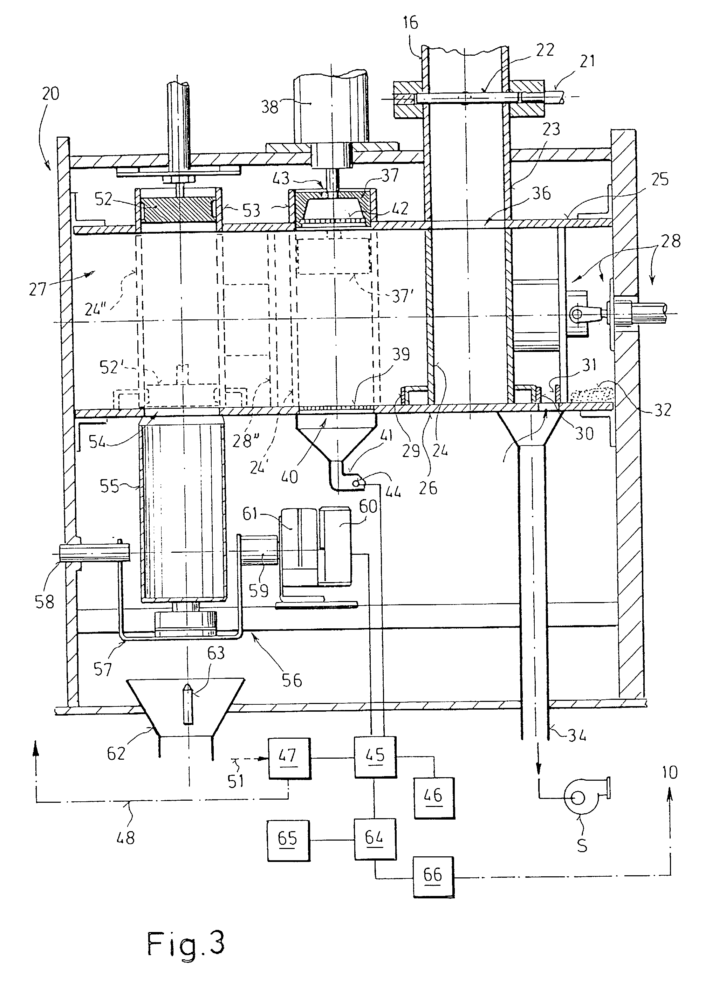

An den Probenehmerabschnitt 16 schliesst das Gehäuse einer Messeinrichtung 20, deren Einzelheiten den Fig. 2 und 3 zu entnehmen sind. Demnach ist die Messeinrichtung 20 an den Rohrleitungsabschnitt 16 angeflanscht, wobei dazwischen ein über eine Stange 21 betätigbarer Absperrschieber 22 vorgesehen sein kann. Wie später noch erläutert wird, wird durch diesen Absperrschieber 22 gesichert, dass das Probematerial stets aus einer vorbestimmten Fallhöhe über einen innerhalb des Gehäuses 20 angeordneten und an den Rohrabschnitt 16 angeflanschten Stutzen 23 in ein darunterliegendes Probengefäss 24 fällt.

Das Gefäss 24 ist innerhalb eines von einer Deckplatte 25 und einer Bodenplatte 26 begrenzten Schieberaumes 27 mit Hilfe eines, beispielsweise mittels eines nicht dargestellten fluidischen Antriebes oder über den Schneckenantrieb eines Motors antreibbaren Schieber 28 in drei verschiedene, strichliert dargestellte Lagen entlang des Bodens 26 verschiebbar.

Vorzugsweise besitzt das Probengefäss 24 an seiner dem Boden 26 zugekehrten Unterseite wenigstens eine Abstreifleiste 29 bzw. 30, durch welche allenfalls über schwer abzudichtende Oeffnungen auf die Oberseite des Bodens 26 gefallene Partikel des zu messenden Schüttgutes zur Seite gestreift werden, um Reibungsstörungen zu vermeiden. Auch der Schieber 28 kann an seiner dem Boden 26 zugewendeten Seite mit einer Abstreifleiste 31 versehen sein, obwohl es an sich auch möglich ist, die Schieberwandung oberhalb des Bodens 26 so weit enden zu lassen, dass sich über dem Boden eine Schlitzöffnung befindet.

Durch diese Abstreifleisten 29 bis 31 wird etwaiges Schüttgut zur Seite (und allenfalls zum Rohr 34) geschoben, wie aus angedeuteten Schüttguthäufchen 32 erkennbar ist. Bevorzugt ist es jedoch, innerhalb des Bodens 26 im Bereiche wenigstens einer Endstellung einer solchen Abstreifleiste (hier die Abstreifleiste 30) eine Öffnung 33 vorzusehen, an die zumindest eine in das Gehäuse 20 mündende, z.B. zur Gutabfuhr 19 (Fig. 1) führende Leitung 34 und allenfalls an eine Absaugeinrichtung s angeschlossen ist. Damit kann die Notwendigkeit periodischer Reinigungen zum Entfernen der Häufchen 32 vermieden werden, indem die Rohrleitung 34 mit ihrer Mündung 33 im Boden 26 für eine Selbstreinigung sorgt. Es versteht sich, dass auch an jenen Stellen, in denen die Häufchen 32 angedeutet sind, der Leitung 34 entsprechende Reinigungsleitungen vorgesehen sein können.

Wie schon erwähnt, kann die Klappe 15 (Fig. 2) zeitabhängig gesteuert sein. Hierzu mag ein Programmsteuerwerk 35 vorgesehen sein, das die Bewegungen der einzelnen Teile über entsprechende Ausgänge 15', 22' und 28' zur Betätigung der Klappe 15, des Absperrschiebers 22 und des Schiebers 28 in der jeweils nötigen zeitlichen Aufeinanderfolge steuert. Auf diese Weise wird zunächst die Klappe 15 kurzzeitig geöffnet, um einen Teilstrom aus der Leitung 14 in die Probennehmerleitung 16 umzuleiten. Sobald die Klappe 15 die Zweigleitung 16 wieder verschlossen hat, wird der Absperrschleber 22 geöffnet, sodass die auf ihm ruhende Partikelmenge über den Rohrstutzen 23 in das Probengefäss 24 fällt. An sich mag es zweckmässig erscheinen, die Öffnungszeit für die Klappe 15 so zu wählen, dass das Probegefäss 24 nicht überfüllt wird. Um es aber mit Sicherheit gänzlich zu fällen, wird diese Zeit vorzugsweise so bemessen, dass das Gefäss 24 stets überfüllt wird, wobei zum Wegräumen des Überschusses die Leisten 29 bis 31 (Fig. 3) vorgesehen sind. Es versteht sich ferner, dass in der Deckplatte 25 in Höhe des ortsfesten Stutzens 23 eine Öffnung 36 vorgesehen ist.

Sobald dies geschehen ist, wird der Schieber 28 betätigt und verschiebt das Probegefäss 24 in die strichliert dargestellte Stellung 24'. An der der Lage 24' entsprechenden Station wird die eigentliche Messung im Sinne von DIN 66126 vorgenommen, d.h. es wird die Schüttgutprobe innerhalb des Probenbehälters 24 unter vorbestimmtem Drucke komprimiert. Zu diesem Zwecke ist an der Oberseite des Probegefässes 24 in seiner Stellung 24' ein Kolben 37 vorgesehen, der über ein Zylinder-Aggregat 38 in seine abgesenkte Stellung 37' unter dem vorbestimmten Drucke bewegbar ist, so dass die Schüttgutprobe unter diesem vorbestimmten Drucke in vorbestimmter Weise zusammengepresst wird.

Nach DIN 66126 muss sodann die Probe von einem Gas vorbestimmter Menge pro Zeiteinheit durchströmt werden, um die Permeabilität bzw. den Durchströmwiderstand zu messen (als Variante ist ein stets gleichbleibendes Gasvolumen denkbar). Im Prinzip wäre dies in einer von der Stellung 24' abweichenden, einem nächsten Bewegungsschritt entsprechenden Stellung möglich, doch kann das Messgerät 20 auch in der dargestellten Weise ausgeführt sein, wobei im Boden 26 eine durch ein Sieb 39 abgedeckte Öffnung 40 vorgesehen ist. Das Sieb 39 hat eine Maschenweite, die klein genug ist, um dem Drucke des Kolbens 37 einen Widerstand entgegenzusetzen und die Partikel der Probe nicht hindurchzulassen.

An diese Öffnung 40 ist nun eine Gaszufuhreinrichtung 41, insbesondere für Luftzufuhr, vorgesehen, um die im Gefässe 24 befindliche Probe zu durchströmen. Prinzipiell könnte die Anordnung auch so getroffen sein, dass anstelle des Siebes 39 ein Abdeckschieber vorgesehen ist, der dann geöffnet wird, wenn der Kolben 37 die Probe bereits zusammengepresst hat und in seine mit vollen Linien dargestellte Lage zurückgekehrt ist (was aber nicht unbedingt erfolgen muss), so dass durch Wegziehen dieses Schiebers die Öffnung 40 für die Gaszufuhr freigegeben wird.

Der Kolben 37 seinerseits besitzt zweckmässig ebenfalls ein relativ engmaschiges Sieb 42, dessen Maschen klein genug sind, um einen wirksamen Druck auf die Probe ausüben zu können, aber gross genug, um das über die Zuleitung 41 herangebrachte und die Probe durchströmende Gas hindurchzulassen. An der Oberseite des Kolbens 37 mag dann wenigstens eine Öffnung 43 vorgesehen sein, durch welche die zur Permeabilitätsmessung verwendete Luft ins Freie strömen kann oder über eine nicht dargestellte Leitung abgeleitet wird, beispielsweise um im Kreislauf wieder der Leitung 41 zugeführt zu werden. Wie schon erwähnt, ist es durchaus möglich, etwa das Sieb 39 oder eine andere Abdeckung (der erwähnte Schieber) an einer anderen Station vorzusehen, die dann selbstverständlich den Kolben 37 nicht aufzuweisen braucht. In diesem Falle mag dann der Kolben 37 ohne die Teile 42 und 43 ausgebildet sein. Innerhalb der Leitung 41 ist ein Sensor 44 an einer an sich beliebigen Stelle angeordnet, der den Druckwiderstand der im Gefäss 24 enthaltenen Probe misst und ein entsprechendes Ausgangssignal an einen Rechner 45 sendet, der von einem Geber 46 die jeweiligen Werte für m und b erhält und darauf entsprechende Signale an eine Regeleinrichtung 47 liefert. Dies kann von einem einzigen Geber 46 aus durch serielle Signale oder über parallele Leitungen erfolgen, oder es ist für die beiden Werte m und b jeweils ein gesonderter Geber vorgesehen.

Ferner errechnet der Rechner 45 aus dem bekannten Volumen des Probengefässes 24 und dem gemessenen Gewicht die Schüttdichte, die besonders dann wichtig ist, wenn das gemahlene Gut anschliessend in Verpackungen vorbestimmter Grösse und vorbestimmten Füllgewichtes eingefüllt werden soll. Auch hier wären Modifikationen denkbar, denn es wäre auch denkbar, dass das Schüttgut mit vorbestimmtem Druck durch den Kolben 37 zusammengepresst und dann an Hand der Kolbenstellung (bei Verwendung eines Kolbenpositionssensors) oder eines Niveausensors für das im Probengefäss 24 befindliche Schüttniveau (das hiezu beispielsweise durchsichtig ausgebildet ist, um eine optische Abtastung zu ermöglichen) das Volumen zu ermitteln.

Ein dem errechneten IST-Wert der Schüttdichte entsprechendes Signal wird also vom Rechner 45 einer Regelstufe 64 zugeführt, die einen Sollwert von einem einstellbaren Sollwertgeber 65 erhält. Daraus wird in der Regelstufe ein die Abweichung vom Sollwert berücksichtigendes Regelsignal gewonnen, das einer Stellsignalstufe 66 zugeführt wird. Diese Stellsignalstufe 66 regelt die Drehgeschwindigkeit des Kompaktierwerkes 11 und damit dessen Kompaktierdruck (falls das Kompaktieren mit Hilfe anderer druckausübender Einrichtungen, wie Druckkolben od.dgl. erfolgt, wird deren Druck in analoger Weise geregelt).

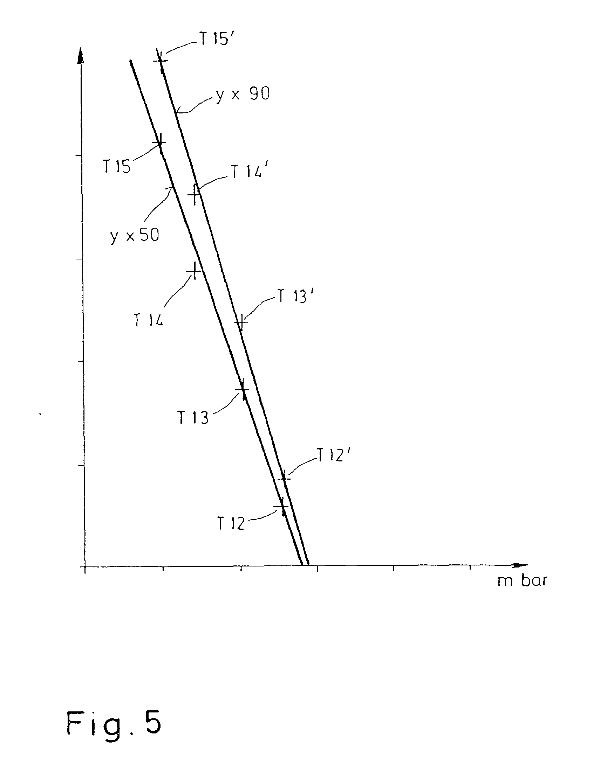

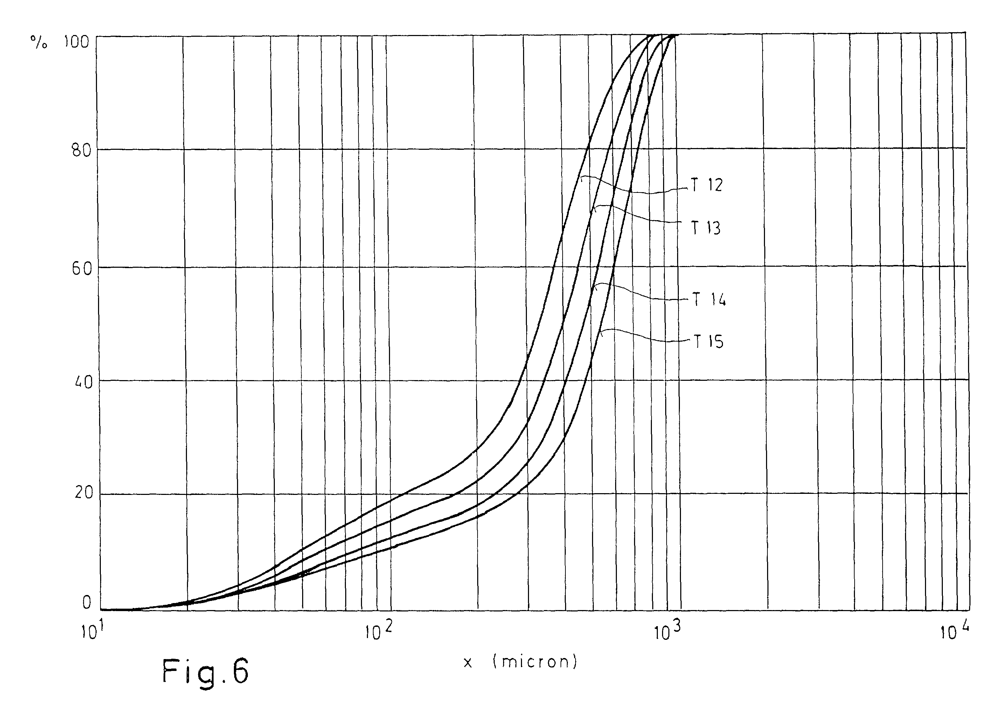

Der Geber 46 kann auf die verschiedenste Weise ausgebildet sein. Im Prinzip kann er nach Art der Station 24'konstruiert sein, um parallel zur Messung an der Stelle 24' eine Vergleichsmessung durchzufahren, wobei die Vergleichsmessung von einer Standardprobe bekannter Partikelgrösse gebildet ist. Falls aber die Eichung von Hand aus vorher vorgenommen wird, kann der Geber 46 aus einer einfachen Eingabetastatur für die betreffenden Werte von m und b für die Maximalpartikelgrösse (siehe die Erläuterungen zu Fig. 3 im Zusammenhang mit Fig. 5 und 6) ausgebildet sein. Es versteht sich, dass, besonders bei Verwendung des Messgerätes 20 ohne die Mühle 1, über den Geber 46 dann dementsprechend die Werte für die durchschnittliche Partikelgrösse eingegeben werden, um diese zu erhalten. Eine weitere Möglichkeit besteht darin, dass der Geber 46 für die Eingabe von Daten eingerichtet ist, die die jeweiligen Werte für m und b erst bestimmen. Ferner könnten die Presskraft des Kolbens 37 (falls diese veränderbar ist) die Probenlänge (falls der Probenbehälter 24 nicht völlig gefüllt wird), der Druck durch die Leitung 41 (falls sich dieser ändern kann) und allenfalls auch die Schüttdichte (falls die Belastung des Kolbens 37 veränderbar ist) eingegeben werden.

Das Ausgangssignal der Rechenstufe 45, die praktisch eine blosse Multiplikationsstufe bekannter Ausbildung darstellt, wird der Regeleinrichtung 47 übermittelt, deren strichpunktierte Ausgangsleitung 48 auch in Fig. 1 zu sehen ist. Denn wenn das Rechenergebnis der Stufe 45 einem Soll-Wert für den Mahlspalt entspricht, so sind zweierlei Vorgangsweisen möglich: entweder es ist ein Positionssensor für die Grösse des Mahlspaltes, beispielsweise für die Relativlage beweglicher Walzenlager 49 gegenüber ortsfesten Walzenlagern 50 vorgesehen, dessen Ausgangssignal über eine strichliert in Fig. 3 gezeigte Leitung 51 als IST-Wert der Regeleinrichtung 47 zugeführt wird. Oder die Regeleinrichtung 47 arbeitet mit einem Inkrementalsystem (z.B. mit einem Schrittmotor) als (nicht dargestelltes) Stellglied, in welchem Falle ihr die jeweilige Stellung der beweglichen Lager 49 bekannt ist. Dies kann z.B. so erfolgen, dass mit dem Einschalten des Betriebes des Mahlwerkes 1 die Walzen 2 bis auf einen Mahlspalt von "0" zusammengefahren werden, um den Null-Punkt zu bestimmen, worauf sie in eine gewünschte und einstellbare Mahlspaltposition wieder auseinanderfahren. Dabei zählt die Regeleinrichtung 47 entweder die Schritte des Schrittmotors oder die Inkrementalschritte eines Inkremental-Weggebers. Da aber häufig die Mahlwalzen 2 eine empfindliche Oberfläche besitzen, ist es bevorzugt, wenn der umgekehrte Weg beschritten wird, indem die Walzen 2 zunächst auf eine definierte, z.B. durch einen Geber oder durch einen Anschlag bestimmte, Offenposition auseinanderfahren und dann erst in die gewünschte Mahlspaltposition zusammengebracht werden.

All diese Massnahmen sind in der Regeltechnik an sich bekannt und bedürfen daher keiner ins Einzelne gehenden Erläuterung. Vielmehr ist verständlich, dass auf diese Weise auch bei der Vermahlung von Trockengütern eine einfache Bestimmung der erzielten Durchschnittsmahlfeinheit und eine entsprechende Nachstellung eines Mühlenaggregates möglich ist. Aus der US-A-3,734,659 ist es beispielsweise bekannt, dass der Mahlspalt bzw. die Feinheit des Mahlgutes auch durch Regelung der Zufuhrmenge durch Beeinflussung der Speisung relativ zu einer gegebenen Umfangsgeschwindigkeit der Mahlwalzen beeinflusse werden kann. Dementsprechend ist auch die Regelung der Walzengeschwindigkeit bei gegebener Zufuhrgeschwindigkeit denkbar.

Wenn nun daher die im Probenbehälter 24 enthaltene Probe bereits in der Stellung 241 gemessen ist, könnte sie zur nächsten Stellung des Schiebers 28, der Stellung 28" bzw. der Stellung 24" des Probenbehälters 24 verschoben werden, wo ein Auswerferkolben 52 in einem ähnlichen Zylinderstutzen 53 geführt ist, wie der Kolben 37. Dieser Auswerferkolben durchfährt den gesamten Probenbehälter 24 und erreicht am Ende seiner Bewegung eine Stellung 52'.

In Fig. 3 ist nun aber noch eine Korrekturmöglichkeit vorgesehen und gezeigt. Wenn nämlich das gemessene Schüttgut aus dem Probenbehälter 24 in der Stellung 24" vom Auswerferkolben 52 durch eine Öffnung 54 der Bodenplatte 26 gedruckt worden ist, kann es in einen Wiegebehälter 55 einer Waage 56 eingeschoben werden, um an dieser Stelle das Gewicht der Probe zu bestimmen. Dieser Wiegebehälter 55 ist an einem etwa U-förmigen Bügel 57 befestigt, dessen Schenkel an Wellenstummeln 58, 59 sitzen. Von diesen Wellenstummeln 58, 59 ist wenigstens einer mit einer Druckmessdose 60 verbunden, über die das Gewicht der Probe bestimmbar ist. Obwohl bei völliger Füllung des Probenbehälters 24 das Gewicht stets gleich sein müsste, können sich geringere Differenzen ergeben, die als Korrekturwert aus der Messeinrichtung 60 in die Recheneinrichtung 45 eingegeben werden.

Es ist jedoch noch ein anderes Vorgehen denkbar, denn einerseits könnte auf den Kolben 37 verzichtet werden, wenn man davon ausgeht, dass die Fallhöhe beim Befüllen des Probenbehälters 24 dazu ausreicht, einen vorbestimmten Druck auf das Material auszuüben und damit eine vorbestimmte Schüttdichte zu erreichen. Zwar könnte für diese Fallhöhe die Länge des Rohrstutzens 23 samt der daran anschliessenden Probenehmerleitung 16 und der darüberliegenden Leitung 14 verwendet werden, doch ergeben sich dabei unter Umständen zu viele Störfaktoren, weshalb es vorteilhaft ist, den Absperrschieber 22 zur Definierung der Fallhöhe anzuordnen. Es könnte dann nach Durchströmung der so ohne den Kolben 37, lediglich aufgrund ihres Eigengewichtes verdichteten Probe mit Luft ein Wiegevorgang im Behälter 55 einsetzen, der allfällige Korrekturen liefert. Andererseits versteht es sich, dass der Wiegebehälter 55 samt Waage 56 auch völlig entfallen kann, etwa wenn das Gewicht in an sich bekannter Weise im Durchlaufverfahren bestimmt wird.

Sobald der Wiegevorgang beendet ist, wird der Wellenstummel 59 von einem mit der Gewichtsmesseinrichtung 60 verbundenen Antrieb 61 an sich beliebiger Art (Dreheiseninstrument, Motor, Elektromagnet, fluidischer Motor) gedreht, wobei der Behälter 55 in eine um 180' gedrehte Lage gelangt und sein Inhalt in einen, zweckmässig zur Einrichtung 19 (Fig. 1) fahrenden Trichter 62 entleert wird. Falls auf eine Waage 56 verzichtet wird, wurde dieser Trichter 62 unmittelbar unter der Öffnung 54 angeordnet sein. Es ist vorteilhaft - insbesondere um das Gewichtsmessergebnis nicht zu verfälschen - im Bereiche des Trichters 62 eine Reinigungseinrichtung für das Behälterinnere des Wiegebehälters 55 vorzusehen. Eine solche Reinigungseinrichtung kann im Prinzip aus einer rotierenden Bürste gebildet sein, doch ist es einfacher und zweckmässiger, hierfür wenigstens eine Fluiddüse 63 vorzusehen, insbesondere eine Druckluftdüse, deren Druckluftzufuhr beispielsweise durch das Programmwerk 35 (Fig. 2) über dessen Ausgangsleitung 63' gesteuert wird.

Es versteht sich, dass auch hinsichtlich der Ausbildung und Anordnung der Waage 56 mannigfaltige Modifikationen denkbar sind, wie etwa die Unterbringung der Messdose 60 zwischen der Unterseite des Behälters 55 und dem Bügel 57, was lediglich den Nachteil besitzt, dass die Zuleitungen beweglich sein müssen. Ferner wurde das Messgerät 20 für eine lineare Verschiebung des Probenbehälters 24 mit Hilfe eines Schiebers 28 erläutert, welch letzterer selbstverständlich nach dem Auswerfen der Probe mittels des Kolbens 52 wieder in die mit vollen Linien dargestellte Lage zurückkehren kann; es wäre aber ebenso denkbar, die einzelnen Stationen 24, 24', 24" entlang einer Kreisbahn vorzusehen und in diesem Falle anstelle eines linear verschiebbaren Schiebers 28 eine Art Revolverkonstruktion vorzusehen, die um eine antreibbare Welle drehbar ist, zu der der Probenbehälter 24 exzentrisch angeordnet wird.

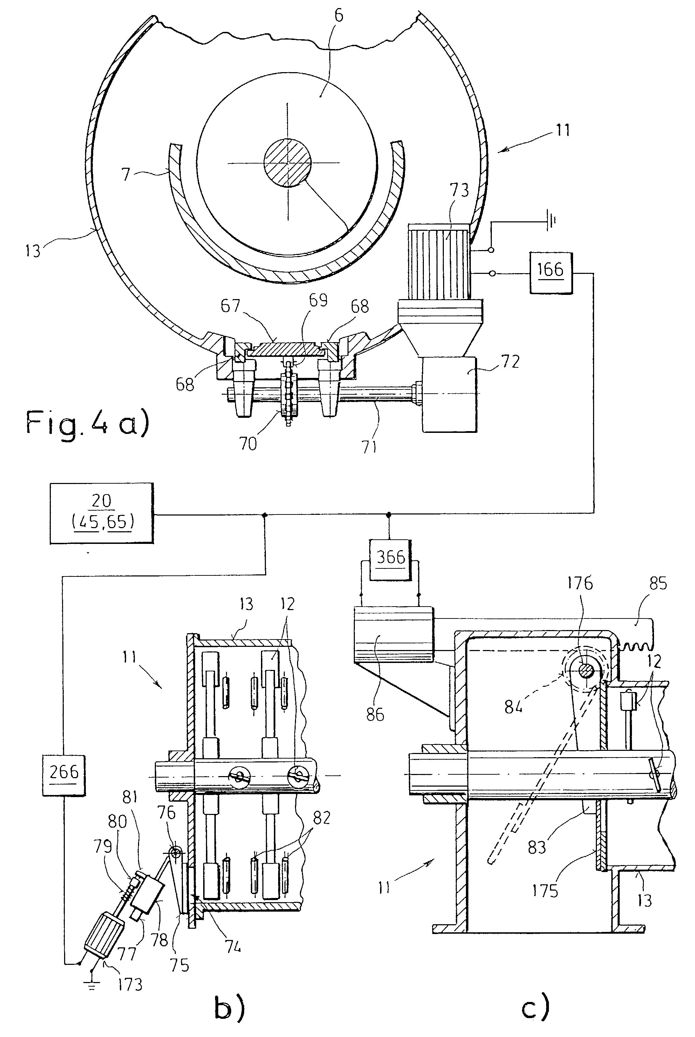

Fig. 4 zeigt in drei, durch die gemeinsame Messeinrichtung 20 bzw. 45, 64 verbundenen, Varianten a) bis c), wie das Ausgangssignal der Messeinrichtung 20 auch noch auf andere Weise verwendet werden kann. Nach Fig. 4 a) ist die Unterseite des Kompaktiergehäuses 13 durch einen Schieber 67 verschlossen, der in Führungen 68 verschiebbar geführt ist und den Auslauf des Kompaktierwerkes 11 mehr oder weniger verschliesst. Zu seiner Verschiebung ist der Schieber 67 an seiner Unterseite mit einer Kette 69 verbunden, die über ein Kettenrad 70 antreibbar ist. Im Prinzip könnte dieser Antrieb auch durch eine Zahnstange am Schieber 67 und ein darin eingreifendes Zahnrad (entsprechend dem Rad 70) erfolgen, doch ist der Kettenantrieb weniger empfindlich und daher bevorzugt.

Das Rad 70 sitzt auf einer Antriebswelle 71, die über ein Getriebe 72 ihren Drehantrieb von einem Stellmotor 73 (vgl. auch Fig. 1) erhält. Es ist aus Genauigkeitsgründen bevorzugt, wenn als Stellmotor 73 ein Schrittmotor verwendet wird. Dieser Schrittmotor 73 wird nun über eine Stellstufe 166 angesteuert, die funktionell der schon beschriebenen Stellstufe 66 entspricht und ihr Regelsignal von den Einrichtungen 45 und 64 erhält.

Im Falle der Fig. 4 b) hingegen ist die Einrichtung 20 mit einer Stellstufe 266 verbunden. Das Kompaktiergehäuse 13 besitzt hier nur eine relativ geringe Auslassöffnung 74, die durch eine um eine Achse 76 schwenkbare Klappe 75 als Gegendruckglied verschlossen ist. Diese Klappe 75 steht unter dem Drucke eines entlang eines Hebels 77 verstellbaren Gewichtes 78. Die Stellung dieses Gewichtes 78, und damit sein Andruckmoment auf die Klappe 75, wird durch die Stellung eines auf einer Gewindespindel verstellbaren Anschlages 80 bestimmt, der mit einem Gegenanschlag 81 am Gewicht 78 zusammenwirkt. Die Spindel 79 ist mit einem Stellmotor 173 verbunden, wobei die ganze Anordnung ähnlich derjenigen ist, wie sie in anderem Zusammenhange bereits in der DE-A-38 39 778 für eine Gewichtsverstellung beschrieben worden ist.

Eine der Fig. 4b ähnliche Konstruktion könnte aber auch noch für einen anderen Zweck eingesetzt werden. Wenn nämlich beispielsweise eine nach unten führende und durch einen Schieber 67 (Fig. 4 a) abdeckbare Öffnung vorgesehen ist, wie dies etwa auch aus Fig. hervorgeht, so könnte eine (feder- oder) gewichtsbelastete Klappe 75 an einer Öffnung 74 zusätzlich vorgesehen sein, um im Falle eines plötzlich auftretenden Staues innerhalb des Gehäuses 11 als Entlastungsklappe zu wirken. In diesem Falle könnte aber wohl eine Regelung der Belastung dieser Klappe entfallen.

Während im Falle der Fig. 4 b) das Kompaktierwerk 11 zusätzlich zu den Paddeln 12 auch vom Gehäuse 13 abstehende Statorwerkzeuge 82 aufweist, die eine Homogenisierung durch das Kämmen mit den Paddeln 12 fördern, fehlen solche Werkzeuge im Falle der Fig. 4 c). Hier ist eine grössere Klappe 175 an einem Hebel 83 befestigt, der mit einer Verstellwelle 176 verbunden ist. An der Verstellwelle 176 sitzt ein, lediglich strichliert angedeutetes Zahnrad 84, mit dem eine Zahnstange 85-in Eingriff steht. Diese Zahnstange 85 wird von einem Zylinderaggregat 86 betätigt, das über eine entsprechende Stellsteuerung 366 angesteuert wird, wobei letztere ihre Regelsignale von der Regeleinrichtung 64 erhält.

Es versteht sich, dass die Verstellung eines Auslauf-Verschlussorganes 67 bzw. 75 oder 175 auch zusätzlich zur Verstellung der Drehzahl des das Kompaktierwerk 11 antreibenden Motors 10 (Fig. 1) erfolgen kann. Beispielsweise geschieht dies nach Art einer Kaskadenregelung, indem erst die eine Regelung (z.B. die des Motors 10) betätigt wird und bei Erreichen der Grenzen des Regelbereiches die jeweils andere (z.B. die des Verschlussorganes). Es kann aber ein rascheres Ansprechen der Regelung und damit eine kürzere Regelzeitkonstante durch gleichzeitiges Ansprechen beider Regelungen erzielt werden.

Es sei bemerkt, dass die Beeinflussung der Kompaktierung auch dadurch vorgenommen werden kann, dass die Speisung des Mahlwerkes entsprechend reguliert wird, wie in Fig. 1 an Hand der strichpunktierten Linie 66 (Ausgang der Regelstufe 66) angedeutet ist. Dies bedeutet aber eine grössere Regelzeitkonstante und wird daher im allgemeinen nicht bevorzugt sein. Eine Ausnahme könnte allerdings die Einbindung dieser Regelungsart in die oben besprochene Kaskadenregelung bilden.

Die Erfindung ist keineswegs auf die Messung relativ spröder Partikel, gegebenenfalls mit nachfolgender Regelung eines Mahlspaltes und/oder Mahldruckes, beschränkt, vielmehr können auch relativ formbare Partikel auf diese Weise gemessen werden. Ein Beispiel mag die Herstellung von Flocken (z.B. Haferflocken, Sojaflocken usw.) auf einem Quetschwalzwerk sein.

Im folgenden wird nun die Messung der Teilchengrösse im Detail beschrieben. Aus der Literatur sind im Prinzip zwei verschiedene Geräte bekannt, mit welchen die spezifische Oberfläche der Schüttung gemessen werden kann.

Das sog. Blaine-Prinzip (DIN 66127) benutzt eine Methode der Durchströmung der Schüttung mit zeitlich veränderlichem Druckabfall und das Lea- und Nurse-Gerät mit zeitlich konstantem Druckabfall.

Beide Messprinzipien dienen zur Bestimmung der spezifischen Oberfläche der Schüttung, und der Erfindung liegt die Erkenntnis zugrunde, dass die spezifische Oberfläche eine Funktion der Teilchengrösse, insbesondere der mittleren, ist.

Eine Messung eines die spezifische Oberfläche bestimmenden Parameters und sein Vergleich mit entsprechenden Eichwerten ergibt daher die Kenntnis der Partikelgrösse.

Im Interesse einer automatisierten fliegenden Messung der Teilchengrösse zum Zweck der Regelung eines Mahlwerks ist es selbstverständlich nicht wünschenswert, von Zeit zu Zeit eine Probeschüttung zu entnehmen und diese einer manuellen Messung in einem der oben erwähnten Geräte zuzuführen, die Messung auszuwerten und eine entsprechende Regelung des Mahlwerks manuell vorzunehmen, sondern all diese Vorgänge sollen automatisch durchführbar sein.

Da die Messung der spezifischen Oberfläche einer Schüttung in beiden genannten Geräten eine genau definierte Schüttungsgeometrie vorschreibt, welche bei automatisch ablaufenden Probenherstellungen nur in bestimmten Grenzen erreicht werden kann, kann es notwendig sein, Korrekturmöglichkeiten vorzusehen, welche die Abweichung der reellen Probengeometrie von der vorgeschriebenen Geometrie zu kompensieren gestatten.

Beispielsweise ist beim Blaine-Prinzip bei vorgegebenem Probenquerschnitt und Höhe die Probe mit einer bestimmten definierten Kraft zu verdichten, um eine die Teilchengrösse repräsentierende Porosität zu erreichen. Bei der Verdichtung mittels eines Kolbens im Messzylinder unter einer definierten Kraft wird jedoch eine Probenhöhe erreicht, welche nicht konstant ist, sondern von der Teilchengrösse selbst, aber auch von anderen Parametern wie Luftfeuchtigkeit u.dgl. abhängt, soferne nicht die Probenmenge zum Zwecke der Konstanthaltung des Volumens verändert wird, wie dies nach Blaine vorgesehen ist.

Es muss daher vorgesehen werden, dass die reelle Probenhöhe bestimmbar ist, damit zusammen mit dem Probengewicht die Porosität errechnet werden kann, welche in die Bestimmungsgleichung der spezifischen Oberfläche

$$Sv = \frac{k}{(1 - epsilon)} \bullet Wurzel \sqrt{\frac{epsilon^3 \cdot t}{eta}}$$

eingeht. Hierbei bedeuten Sv die spezifische Oberfläche pro Volumeneinheit, epsilon die Porosität, eta die Viskosität der Luft und t die Durchströmungsdauer eines definierten Luftvolumens durch die Schüttung. k ist ein, der spezifischen Systemgeometrie entsprechender Proportionalitätsfaktor.

Bei Durchführung einer Durchströmungsmessung wird z.B. t, die Dauer der Durchströmung der Probe von einem bestimmten Luftvolumen, ermittelt und über die obige Gleichung die spezifische Oberfläche, und daraus die Partikelgrösse in einem Mikroprozessor berechnet. Hierzu kann eine formelmässige Berechnung von Sv und/ oder eine Bestimmung der Partikelgrösse durch Vergleich der spezifischen Oberfläche mit einer Eichkurve erfolgen. Die Bestimmung der Eichkurve erfolgt mittels identisch ablaufenden Durchströmungsmessungen von Proben, deren mittlere Partikelgrössen bekannt sind, und die unter Aufwendung der gleichen Kraft verdichtet worden sind und eine definierte Probenhöhe aufweisen.

Wie weiter oben erwähnt wurde, muss für die Kompensierung der Abweichung der reellen Probenhöhe von der vorgeschriebenen Höhe, allenfalls Sorge getragen werden. Dies kann nach Kenntnisnahme der reellen Probenhöhe auf mathematischem Wege direkt während der Berechnung im Mikroprozessor erfolgen, wobei die Grösse des Proportionalitätsfaktors k entsprechend korrigiert wird. Die tatsächliche Probenhöhe kann beispielsweise über den Weg des Verdichtungskolbens gemessen werden, wobei die Abweichung der reellen Endstellung des Verdichtungskolbens von der angestrebten Endstellung als Korrekturwert benutzt werden kann. Die Bestimmung der reellen Endstellung des Verdichtungszylinders kann durch Wegmessung oder Positionsmessung evtl. mechanischer, elektrischer oder optischer Weise erfolgen. Selbstverständlich ist es auch möglich, den Füllstand im Probenzylinder nach Verdichtung direkt zu messen. In der Praxis wird man eine Variante wählen, die bezüglich Genauigkeit, Geschwindigkeit und Kosten den übrigen Bedingungen angepasst ist.

Die Fig. 1 bis 3 zeigen, wie oben erwähnt, eine erfindungsgemässe Messeinrichtung, welche fähig ist, in gewünschten Zeitabständen Probenzylinder zu füllen, zu komprimieren und einer Messstation zuzuführen, in der ein bestimmtes Luftvolumen durch die Probe geschickt wird.

Die oben angegebene Gleichung gilt für eine Messung nach dem Blaine-Verfahren mit variablem Druck und ist hier als Beispiel zu verstehen. Selbstverständlich können die Durchströmungsmessungen auch auf andere Art und Weise durchgeführt werden, beispielsweise mit konstantem Druckabfall, wobei die Luftmenge in der Zeiteinheit bestimmt wird oder der Druckabfall bei erzwungener Durchströmung eines definierten Gasvolumens in bestimmter Zeit gemessen wird, wie nach dem Prinzip von Lea- und Nurse.

Wie weiter oben erwähnt, kann diese Eichkurve gegebenenfalls durch eine einzelne Referenzmessung gewonnen werden, da es sich herausgestellt hat, dass der Zusammenhang von Strömungswiderstand und Partikelgrösse in weiten Bereichen linear und proportional ist. Dies wird an Hand der nachfolgenden Tabelle deutlich, die die Messungen an Hand von vier Proben T12 bis T15 veranschaulicht.

Diese Proben wurden auf Grund der Messung des Druckabfalles mit einem Gerät nach Fig. 3 auf Grundverschiedener Vorverdichtungen durch den Kolben 37 berechnet und ihre tatsächliche, auf herkömmlichem Wege bestimmte Partikelgrösse damit verglichen. Fig. 5 zeigt, dass sich dabei eine lineare Korrelation zwischen dem Druckabfall x und der Partikelgrösse für $y_{x50}$ und $y_{x90}$ ergibt, wobei es sich bei $y_{x50}$ um einen der durchschnittlichen Mahlfeinheit entsprechenden Wert handelt, bei $y_{x90}$ praktisch um einen der Maximalpartikelgrösse entsprechenden Wert. Die Tabelle veranschaulicht sehr gut, dass der Korrelationskoëffizient R eine weitgehende Entsprechung mit Werten zwischen -0,989 und -0,997 angibt. Dabei wurde der Ordnung halber auch der Korrelationskoëffizient R (L) für den Zusammenhang zwischen dem Mahlspalt und den durchschnittlichen (x50) und maximalen (x90) Partikelgrössen berechnet und findet sich in der Grössenordnung von 0,997 bis 0,999.

Die Rückrechnung auf die Mahlfeinheit erfolgt nach einer linearen Regressionsrechnung

$$y = m \cdot x + b$$

wobei

y    der gesuchte Wert entweder für jene Partikelgrösse ist, die der durchschnittlichen Mahlfeinheit entspricht ($y_{x50}$) oder annähernd der maximalen Partikelgrösse ($y_{x90}$);

m    ist ein dem Anstiegswinkel der Geraden für $y_{x50}$ bzw. $y_{x90}$ in Fig. 5 entsprechender Wert;

x    der Druckabfall in mbar; und

b    bedeutet den Wert am Schnittpunkt der Geraden $y_{x50}$ bzw. $y_{x90}$ der Fig. 5 mit der y-Achse, d.h. die Entfernung dieses Schnittpunktes vom Nullpunkt.

Man sieht, dass der Anstiegswinkel, d.h. der Wert von m je nach der gesuchten Partikelgrösse ($y_{x50}$ oder $y_{x90}$), unter der Voraussetzung gleichen Gasdruckes durch die Probe im Probengefäss 24, verschieden ist. Dagegen hängt b im wesentlichen vom Produkt ab, insoferne bei gleicher Steigung (Wert m) der Abstand der Korrelationsgeraden von der y-Achse für den Schnittpunkt mit dieser letzteren massgebend ist. Selbstverständlich liesse sich die Berechnung auch auf andere Weise durchführen, doch erscheint die oben beschriebene als die für praktische Zwecke günstigste.

Grundsätzlich liessen sich auch nicht-lineare Regressionsrechnungen anwenden, um zum gewünschten Ergebnis zu kommen, doch ist der Zusammenhang, wie Fig. 5 deutlich zeigt, so weitgehend linear, dass mit der obigen Berechnungsmethode das Ziel auf vereinfachtem Wege erreicht wird, was sich letztlich auch in einer Vereinfachung des Rechners 45 auswirkt.

Die Partikelgrössenverteilung der vier Proben T12 bis T15 ist der Fig. 6 zu entnehmen, in der an der vertikalen Achse praktisch die Prozentwerte aufgetragen sind, auf der x-Achse die Partikelgrösse in micron, so dass sich bei 50% die durchschnittliche Feinheit (gemessen mit einer herkömmlichen Methode) feststellen lässt, bei 90% etwa (nicht ganz) die maximale Partikelgrösse.

Tabelle    Uebersicht der Messergebnisse und Werte der linearen Regressionsrechnung $y = m \cdot x + b$
R = Korrelationskoeffizient

| Probe Nr. | Mahlspalt L (mm) | Partikelgrösse (micron) | | Druckabfall p (mbar) bei verschiedenen Vorverdichtungen | | |
|---|---|---|---|---|---|---|
| | | x50 | x90 | F = 0 N | F = 250 N | F = 500 N |
| T 12 | 0.20 | 333 | 600 | 51 | 128 | 159 |
| T 13 | 0.24 | 403 | 690 | 41 | 96 | 118 |
| T 14 | 0.27 | 472 | 766 | 29 | 65 | 80 |
| T 15 | 0.30 | 546 | 845 | 21 | 48 | 55 |
| R (x90) | | | | -0.997 | -0.993 | -0.996 |
| R (x50) | | | | -0.996 | -0.989 | -0.993 |
| R (L) | | 0.997 | 0.999 | | | |

**Patentansprüche**

1. Regelvorrichtung für ein Mahlwerk mit mindestens zwei miteinander einen Mahlspalt begrenzenden Walzen (2) und einer Stelleinrichtung (48, 49) zur Einstellung mindestens eines den Mahlspalt bestimmenden Parameters, dadurch gekennzeichnet, dass an den Ausgang eines Kompaktierwerkes (11) für das Mahlwerk eine Messeinrichtung für Volumen und Gewicht einer Probe angeschlossen ist, deren Messignale einer Recheneinrichtung (45) zur Errechnung der Schüttdichte zuführbar sind, deren Ausgangssignale einer Regeleinrichtung (64) zum Vergleich mit einem Sollwert übermittelbar sind, an welch letztere eine Stelleinrichtung (10, 11, 66; 73, 166; 173, 266; 366, 86) zur Veränderung der Schüttdichte angeschlossen ist.

2. Vorrichtung nach Anspruch 1, dadurch gekennzeichnet, dass die Stelleinrichtung (10, 11, 66; 73, 166; 173, 266; 366, 86) ein Kompaktierorgan (11) aufweist, wobei an diesem Kompaktierorgan (11) zur Veränderung der Schüttdichte die Antriebsgeschwindigkeit bzw. der Kompaktierdruck und/oder der Auslassquerschnitt veränderbar ist,

3. Vorrichtung nach Anspruch 2, dadurch gekennzeichnet, dass zur Veränderung des Auslassquerschnittes ein Verschlussorgan (67; 75; 175), wie eine Auslassklappe (75; 175), ein Auslassschieber (67) od.dgl vorgesehen ist, und wobei die Stelleinrichtung einen Antrieb (73; 173; 86) für die Verstellung dieses Verschlussorganes (67; 75; 175) aufweist.

4. Vorrichtung nach einem der Ansprüche 1 bis 3, dadurch gekennzeichnet, dass sie eine Einrichtung (16, 21) zur, insbesondere diskontinuierlichen, Entnahme von Schüttgutproben eines kontinuierlichen Schüttgutflusses, eine Manipulationseinrichtung (28a) zum Transport eines zur Füllung mit Schüttgut dienenden Probengefässes (24) aufweist, und mit einer Mehrzahl von Stationen (24', 24"), wovon eine Station eine Füllstation und eine andere Station eine Messstation ist, wobei die in der Messstation ermittelten Werte der Recheneinrichtung (45) zuführbar sind.

5. Vorrichtung nach Anspruch 4, dadurch gekennzeichnet, dass eine der Stationen mit einer, einen Kolben (37) aufweisenden Verdichtungseinrichtung ausgestattet ist, mittels welchem die Schüttgutprobe verdichtet wird.

6. Vorrichtung nach Anspruch 4 oder 5, dadurch gekennzeichnet, dass die Manipulationseinrichtung (28a) eine Verschiebeeinrichtung (28) für das Probengefäss (24) sowie eine Bodenplatte (26) aufweist, auf welcher das unten offene Probengefäss (24) mittels des Verschiebekolbens (28) von einer der Stationen (241, 24") zur anderen verschiebbar ist;

7. Vorrichtung nach einem der Ansprüche 4 bis 6, dadurch gekennzeichnet, dass an einer der Stationen (24"), insbesondere an der letzten, eine Wiegeeinrichtung (55 - 61) angeordnet ist.

Fig.1

Fig. 2

Fig.3

Fig. 4 a)

b)

c)

Fig. 5

Fig.6

EP 0 891 935 A1

**Europäisches Patentamt**

# EUROPÄISCHER RECHERCHENBERICHT

Nummer der Anmeldung

EP 98 20 2206

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | DE 23 34 396 A (HAVER & BOECKER) 30. Januar 1975 * das ganze Dokument * | 1-3 | B65G65/46 B65B1/44 |
| A | FR 2 440 576 A (CAMBIER B) 30. Mai 1980 * Seite 5, Zeile 4 - Seite 10, Zeile 26; Abbildungen 1-3 * | 1,2,4,7 | |
| A | NL 8 801 521 A (KOUTSTAAL W) 2. Januar 1990 * Seite 2, Zeile 10 - Seite 3, Zeile 15; Seite 5, Zeile 9 - Seite 6, Zeile 27; Anspruch 6; Abbildungen 1-3 * -& DATABASE WPI Section PQ, Week 9004 Derwent Publications Ltd., London, GB; Class Q31, AN 90-028286 XP002083736 * Zusammenfassung * | 1 | |
| A | FR 2 617 287 A (SERMA) 30. Dezember 1988 * Zusammenfassung; Abbildung 2 * | 1 | RECHERCHIERTE SACHGEBIETE (Int.Cl.6) B65G B65B |

Der vorliegende Recherchenbericht wurde für alle Patentansprüche erstellt

| Recherchenort | Abschlußdatum der Recherche | Prüfer |
|---|---|---|
| BERLIN | 9. November 1998 | Johnson, K |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument
......................................................................
& : Mitglied der gleichen Patentfamilie, übereinstimmendes
   Dokument

EPO FORM 1503 03.82 (P04C03)